# EUROPEAN PATENT APPLICATION

(11) **EP 4 445 840 A1**
(43) Date of publication of application: **16.10.2024**
(21) Application number: 22903071.3
(22) Date of filing: 31.10.2022
(51) Int. Cl.: A61B 5/1486, A61B 5/145, A61B 5/00, A61B 5/1468

(54) **BLOOD GLUCOSE MONITORING CIRCUIT AND MEDICAL DEVICE**

(30) Priority: 07.12.2021 CN 202111484638
(71) Applicant: Shanghai Microport Lifesciences Co., Ltd., Shanghai 201318 (CN)
(72) Inventor: HU, Junfeng, Shanghai 201318 (CN); ZHANG, Zijie, Shanghai 201318 (CN); WANG, Biwen, Shanghai 201318 (CN); CHEN, Xun, Shanghai 201318 (CN); QIU, Dan, Shanghai 201318 (CN)
(74) Representative: Patentship Patentanwaltsgesellschaft mbH
(86) International application number: PCT/CN2022/128629
(87) International publication number: WO 2023/103647

(57) **Abstract**

A blood glucose monitoring circuit and a medical device are disclosed. In the blood glucose monitoring circuit, power-on of a transmitter module (120) is controlled by a magnetically controlled switch (122), allowing automatic start of blood glucose monitoring of a user once a blood glucose monitoring device incorporating the blood glucose monitoring circuit is worn on the user. The magnetically controlled switch (122) is turned on or off under the control of a magnet (111), minimizing the influence of muscle movements or other body activities. Power loss of the transmitter module (120) is minimized during operation of a sensor module (110) and the transmitter module (120), dispensing with the need for restart and reestablishment of a wireless connection between the transmitter module (120) and a terminal device, which is otherwise required. Therefore, the blood glucose monitoring circuit is more reliable and contributes to reducing system power consumption. The saved power can extend the battery life of the transmitter module. The medical device includes the blood glucose monitoring circuit.

## Description

### TECHNICAL FIELD

This application relates to the field of medical devices and, in particular, to a blood glucose monitoring (BGM) circuit and a medical device.

### BACKGROUND

Blood glucose monitoring (BGM) forms an important part of diabetes management. BGM results are helpful in assessing the severity of glucose metabolism disorders in patients with diabetes, prescribing glucose-lowering strategies for them, reflecting the efficacy of such therapy regimens and providing guidance in the adjustment of the therapy regimens. Blood glucose concentration is related to exercise, diet, medication and many other factors. Traditional BGM relies on analysis of blood samples collected from finger tips. However, it is impractical for this method to monitor the blood glucose level in a patient throughout the day, and there tend to be time intervals in which the patient is out of monitoring. To overcome this, a dynamic BGM product has been developed in recent years, which are capable of continuous glucose monitoring throughout the day and dynamic monitoring of the blood glucose level in a patient with diabetes.

Despite the ability to continuously monitor the blood glucose level in real-time, the existing dynamic BGM devices are problematic in several respects. Firstly, the existing dynamic BGM devices include a transmitter which is activated by acting on a switch to connect it to a power supply bus. As the switch is a metal pad provided on a base of a blood glucose sensor, the transmitter may be accidentally disconnected from the power supply bus due to muscle movements or other body activities and lose power as soon as the disconnection happens. Restarting the transmitter and reestablishing a wireless connection will increase system power consumption. Therefore, their stability is poor. Secondly, the transmitter of the existing device has a large number of electronic circuit modules, which result in not only a complicated hardware system but also significant overall power consumption of the transmitter. Consequently, a rechargeable lithium battery for the transmitter runs out before the blood glucose sensor fails. Although a new connection can be established to the blood glucose sensor after the battery is recharged, the BGM data stream has to be interrupted, and an increase is caused in operational burden of the patient. Moreover, it is not a good idea to use a larger capacity battery because it would make the transmitter bulkier and aggravate the patient's foreign body sensation.

Therefore, there remains a challenge to improve the service life and reliability of the transmitter while not increasing its volume or using a larger capacity battery, in order to achieve the dynamic blood glucose monitoring in a convenient and inexpensive way.

### SUMMARY

This application provides a BGM circuit incorporating a cost-saving transmitter with an extended service life and enhanced reliability. A medical device is also provided.

In one aspect of this application, there is provided a BGM circuit including:
a sensor module including a blood glucose sensor, the blood glucose sensor configured to detect a blood glucose level in a human body and output a corresponding electrical signal; and a transmitter module detachably connected to the sensor module, the transmitter module including a battery, a plurality of electricity-consuming components and a magnetically controlled switch disposed between the battery and the plurality of electricity-consuming components, the transmitter module configured to apply an excitation voltage to the blood glucose sensor, collect the electrical signal output from the blood glucose sensor and process the electrical signal to generate a measurement value reflecting the blood glucose level in the human body, wherein the sensor module further includes a magnet, and when the magnetically controlled switch approaches the magnet to a predetermined extent, a circuit of the magnetically controlled switch is closed, and the plurality of electricity-consuming components are responsively powered on.

In some embodiments, the plurality of electricity-consuming components in the transmitter module include:
a sensor excitation unit for generating the excitation voltage applied to the blood glucose sensor; a sensor signal conditioning unit for collecting the electrical signal output from the blood glucose sensor, which is an analog current signal, and converting the analog current signal to a corresponding analog voltage signal; and a processor unit for receiving the analog voltage signal output from the sensor signal conditioning unit, generating the measurement value reflecting the blood glucose level in the human body by processing the analog voltage signal and sending the measurement value to a terminal device.

In some embodiments, the processor unit includes:
an analog-to-digital converter (ADC) for amplifying the analog voltage signal output from the sensor signal conditioning unit and converting the amplified signal into a linearly corresponding digital voltage signal; a filter for generating the measurement value reflecting the blood glucose level in the human body by performing filtering and smoothing processing on the digital voltage signal output from the ADC; and a wireless communication module for transmitting the measurement value to the terminal device.

In some embodiments, the sensor excitation unit and the sensor signal conditioning unit are provided by a dual-channel operational amplifier (op-amp) chip.

In some embodiments, the plurality of electricity-consuming components in the transmitter module include a bias voltage control unit and a sensor excitation unit, the bias voltage control unit configured to output, in different time intervals during operation of the blood glucose sensor, respective control signals to the sensor excitation unit to adjust the excitation voltage applied to the blood glucose sensor.

In some embodiments, the bias voltage control unit includes a voltage divider chip, a first voltage-dividing circuit and a second voltage-dividing circuit, both the first voltage-dividing circuit and the second voltage-dividing circuit connected to the voltage divider chip, the voltage divider chip switchable between the first voltage-dividing circuit and the second voltage-dividing circuit by means of an analog switch so that one of the first voltage-dividing circuit and the second voltage-dividing circuit divides a voltage and produces a corresponding divided voltage which is then output to the sensor excitation unit.

In some embodiments, the excitation voltage is configured as: relative to an activation time interval for the blood glucose sensor is implanted into the human body, a value of the excitation voltage decreases after the blood glucose sensor is implanted into the human body upon the expiry of the activation time interval.

In some embodiments, the plurality of electricity-consuming components in the transmitter module further include: a voltage regulator connected to the bias voltage control unit, the voltage regulator configured to generate a reference voltage based on an output from the battery and provide the reference voltage to the bias voltage control unit as a reference voltage source for voltage division; and/or a battery capacity measurement unit for measuring remaining capacity of the battery which is fed back to the processor unit.

In some embodiments, the transmitter module includes a primary output circuit and a secondary output circuit, the primary output circuit and the secondary output circuit are connected in sequence to an output of the battery, the primary output circuit including an output node arranged downstream of the magnetically controlled switch and upstream of the plurality of electricity-consuming components, an output voltage of the primary output circuit exceeding an output voltage of the secondary output circuit, wherein the processor unit and the battery capacity measurement unit are both connected to the output voltage of the primary output circuit for power-on, and the sensor signal conditioning unit, the bias voltage control unit and the voltage regulator are all connected to, and powered by, the output voltage of the secondary output circuit.

In some embodiments, the battery is implemented as a single-use disposable battery.

In another aspect of this application, there is provided a medical device including the BGM circuit as defined above.

### Beneficial Effects

In the BGM circuit of this application, the magnetically controlled switch based power-on strategy enables automatic start of BGM of a user once it is worn on the user, without switch contacts arranged on the transmitter and a base of the sensor. Thus, enhanced reliability is achieved. The BGM circuit also has the advantages as follows:
Firstly, the bias voltage control unit allows the excitation voltage to be adjusted. In this way, taking into account the service life of a BGM device incorporating the BGM circuit, electrochemical reactions around a probe of the sensor can be initiated and reach equilibrium more quickly, shortening an initial adaptation period required by the BGM device and allowing it to provide measurements faster. Moreover, glucosidases are allowed to react at a reasonable speed which avoids them from being consumed too fast to ensure that the blood glucose sensor can effectively operate long enough. Besides, interference from other components of the interstitial fluid can be minimized.

Secondly, the use of the single-use disposable battery dispenses with a power supply management module and a charging interface, which would be otherwise necessary for the transmitter if a rechargeable battery is used. In this way, a reduced volume of the transmitter module and increased ease of user operation can be achieved.

Thirdly, the use of the processor that incorporates the ADC and the wireless communication module enables a higher degree of circuit integration.

Fourthly, the sensor excitation unit and the sample conditioning unit are provided by a dual-channel op-amp chip, and a current limiting means is provided at a power supply terminal of the chip. Both these can help reduce power consumption of the chip and prolong the battery life of the transmitter module. As a result, the service life of the transmitter module can be extended expectedly to a period of time that is more than one times the service life of the blood glucose sensor (which is a single-use disposable component) without increasing the volume of the transmitter module 120 (i.e., without using a larger capacity battery). Moreover, the battery can be easily replaced when it runs out. Therefore, dynamic blood glucose monitoring can be achieved in a convenient, reliable and inexpensive way.

The medical device of this application that includes the BGM circuit has similar advantages with the BGM circuit.

### BRIEF DESCRIPTION OF THE DRAWINGS

Fig. 1 is a schematic diagram showing the structure of a BGM circuit according to an embodiment of this application.
Fig. 2 is a schematic circuit diagram of a primary output voltage generator in a BGM circuit according to an embodiment of this application.
Fig. 3 is a schematic circuit diagram of a secondary output voltage generator and a voltage regulator in a BGM circuit according to an embodiment of this application.
Fig. 4 is a schematic circuit diagram of a blood glucose sensor, a sensor excitation unit, a sensor signal conditioning unit and a bias voltage control unit in a BGM circuit according to an embodiment of this application.
Fig. 5 is a schematic circuit diagram of a processor unit and a sensor signal conditioning unit in a BGM circuit according to an embodiment of this application.
Fig. 6 is a schematic circuit diagram of a battery capacity measurement unit in a BGM circuit according to an embodiment of this application.

In these figures, 110-sensor module; 111-magnet; 112-blood glucose sensor; 120-transmitter module; 121-battery; 122-magnetically controlled switch; 123-sensor excitation unit; 124-sensor signal conditioning unit; 125-processor unit; 126-bias voltage control unit; 127-voltage regulator; 128-battery capacity measurement unit.

### DETAILED DESCRIPTION

Blood glucose monitoring (BGM) circuits and medical devices constructed in accordance with specific embodiments of this application will be described below in greater detail with reference to the accompanying drawings. From the following description, advantages and features of the application will become more apparent. It would be appreciated that the figures are provided in a very simplified form not necessarily drawn to exact scale for the only purpose of facilitating easy and clear description of the embodiments.

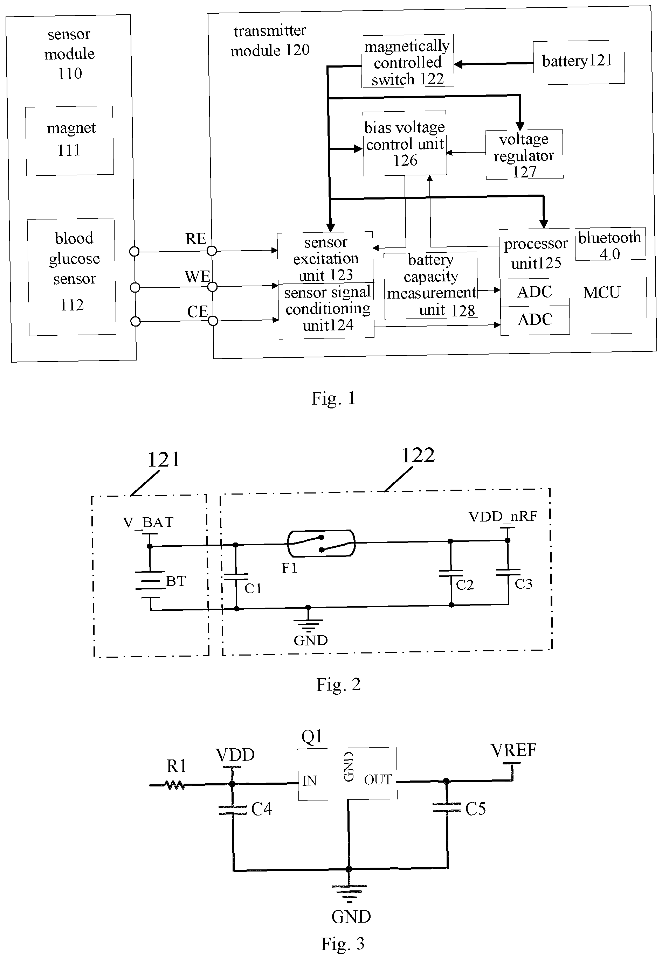

Fig. 1 is a schematic diagram showing the structure of a BGM circuit according to an embodiment of this application. Referring to Fig. 1, an embodiment of this application relates to a BGM circuit including a sensor module 110 and a transmitter module 120, which are detachably connected to each other.

The sensor module 110 includes an implantable blood glucose sensor 112, the implantable blood glucose sensor 112 is implantable and is configured to, when implanted in the body of a human user, detect a blood glucose level in the human body and output a corresponding electrical signal. A probe of the blood glucose sensor 112 detects redox reactions of glucose that occur in the subcutaneous interstitial and intercellular fluids in the user's body and produce analog current signals. The blood glucose sensor 112 may adopt any suitable sensor structure known in the art.

The transmitter module 120 is configured to apply an excitation voltage to the blood glucose sensor 112 and collect an output electrical signal from the blood glucose sensor 112. The electrical signal is processed into a measurement value reflecting a blood glucose level in the human body, which is then sent to a terminal device. The transmitter module 120 includes a battery 121 and a plurality of electricity-consuming components all connected to the battery 121. The transmitter module 120 is connected to the sensor module 110 so that the blood glucose sensor 112 in the sensor module 110 is connected to at least one of the electricity-consuming components in the transmitter module 120 and hence to the battery 121. The battery 121 is configured to supply energy (i.e., electrical power) to the plurality of electricity-consuming components in the transmitter module 120, as well as being configured to supply energy (i.e., electrical power) to the blood glucose sensor 112 when it is connected to the transmitter module 120.

In embodiments of this application, the sensor module 110 further includes a magnet 111. The magnet 111 may be implanted along with the blood glucose sensor 112 under the skin of the user in need of BGM. Additionally, the magnet 111 may be provided on a base of the blood glucose sensor 112. The transmitter module 120 further includes a magnetically controlled switch 122 which is disposed at an output of the battery 121, the magnetically controlled switch 122 controls the output of the battery 121. Specifically, when a circuit of the magnetically controlled switch 122 is closed, the electricity-consuming components in the transmitter module 120 are powered by the battery 121 and become operable. When the circuit of the magnetically controlled switch 122 is opened, the electricity-consuming components in the transmitter module 120 become inoperable. The magnetically controlled switch 122 may include a normally open reed switch or a normally closed reed switch. The magnet 111 in the sensor module 110 is configured to trigger turn-on of the magnetically controlled switch 122. Specifically, when the magnetically controlled switch 122 approaches the magnet 111 and reaches a predetermined degree of proximity thereto (i.e., when it moves into a predetermined range around the magnet 111), the circuit is closed under the action of a magnetic field produced by the magnet 111. When the magnetically controlled switch 122 moves away from the magnet 111 and is located at a distance therefrom, which exceeds a distance corresponding to the predetermined degree of proximity, it is no longer acted upon by the magnet 111 and the circuit of the magnetically controlled switch 122 is opened. In particular, in order to initiate a BGM process, the sensor module 110 is implanted into the human body, the transmitter module 120 is connected to the sensor module 110 by associated contacts. Moreover, the magnetically controlled switch 122 approaches the magnet 111 in the sensor module 110 and the circuit of the magnetically controlled switch 122 is closed, enabling the battery 121 to supply power to the electricity-consuming components in the transmitter module 120 and the blood glucose sensor 112 in the sensor module 110 and thereby starting the BGM process.

In the above discussed BGM circuit, power-on of the transmitter module 120 is controlled using the magnetically controlled switch 122. In this way, a BGM device incorporating the BGM circuit can automatically initiate a BGM process once it is worn on the user, without switch contacts arranged on the transmitter module 120 and the base of the blood glucose sensor. As the magnetically controlled switch 122 is turned on or off only under the action of the magnetic field produced by the magnet 111, the influence of muscle movements or other body activities on the operation of the sensor module 110 and the transmitter module 120 is minimized. It is less likely for the transmitter module 120 to lose power during operation for such reasons, dispensing with the need for restart and reestablishment of a wireless connection between the transmitter module 120 and the terminal device, which may be otherwise required. Therefore, the BGM circuit is more reliable and contributes to reducing system power consumption. The saved power can extend the battery life of the transmitter module 120 and hence the shelf life of a blood glucose detector product in which the BGM circuit is employed.

The battery 121 in the transmitter module 120 may be implemented as a single-use disposable battery. In this way, a power supply management module and a charging interface, which would be otherwise necessary if a rechargeable battery is used, are not needed, and a reduced volume of the transmitter module 120, lower power consumption and increased ease of user operation can be achieved. For example, the battery 121 is selected as a lithium manganese coin cell with a power rating of 70 mAh. The battery 121 may be placed in a dedicated battery case for easy replacement.

The transmitter module 120 may be provided as a package encapsulated by a biocompatible material, in which the battery 121, the magnetically controlled switch 122 and the electricity-consuming components are all received. The package is encapsulated so that only the contacts for connecting it to the sensor module 110 are exposed. Since the magnetic field can pass through the material that encapsulates the package, exposure of the magnetically controlled switch 122 is unnecessary. Correspondingly, electrode terminals are provided on the sensor module 110, each of which corresponds to a respective one of the contacts exposed at the surface of the transmitter module 120. The electrode terminals are connected to the blood glucose sensor 112 in the sensor module 110 to drive the operation of the blood glucose sensor 112. The sensor module 110 is implanted so that the electrode terminals on the sensor module 110 are exposed and thus can be connected to the contacts on the transmitter module 120. The electrode terminals are connected to the respective contacts by suitable connecting means. As an example, the contacts on the surface of the transmitter module 120 for connection with the sensor module 110 include a reference electrode contact RE, a working electrode contact WE and a counter electrode contact CE, and three electrode terminals corresponding to the three respective contacts are provided on the sensor module 110.

Optionally, it may be configured that the battery 121 provides different power supply voltages to the electricity-consuming components in the transmitter module 120. This can reduce power consumption by narrowing a dynamic operation range of a chip in the transmitter module 120. Fig. 2 is a schematic circuit diagram of a primary output voltage generator in the BGM circuit according to an embodiment of this application. Fig. 3 is a schematic circuit diagram of a secondary output voltage generator and a voltage regulator in the BGM circuit according to an embodiment of this application. Reference is first made to Fig. 2. The transmitter module 120 in the BGM circuit includes a primary output circuit and a secondary output circuit, which are connected in sequence to the output of the battery 121 (BT). An output node of the primary output circuit is arranged downstream of the magnetically controlled switch 122 and upstream of the plurality of electricity-consuming components. An output voltage of the battery 121 is denoted as V_BAT. The magnetically controlled switch 122 is connected to the battery 121, and the circuit of the magnetically controlled switch 122 includes capacitors C1, C2, C3 and a small normally open reed switch F1. The output power supply voltage of the battery 121 is present at the output node of the primary output circuit and is referred to hereinafter as a "first output voltage" denoted as VDD_nRF. Referring to Fig. 3, in this embodiment, the output node of the primary output circuit is connected to the secondary output circuit. The secondary output circuit includes a current limiting resistor R1 connected to the output node of the primary output circuit. The first output voltage is limited by the current limiting resistor R1, resulting in an output voltage of the secondary output circuit, which is referred to hereinafter as a "secondary output voltage" denoted as VDD. In other words, the first output voltage VDD_nRF and the second output voltage VDD are generated by the battery 121 and the primary and secondary output circuits, and it may be configured that each of the electricity-consuming components in the transmitter module 120 is provided with one of the first output voltage VDD_nRF and the second output voltage VDD as a power supply. For example, as detailed below, among the plurality of electricity-consuming components in the transmitter module 120, a processor unit 125 and a battery capacity measurement unit 128 are both connected to the first output voltage VDD nRF for power-on, and a sensor signal conditioning unit 124, a bias voltage control unit 126 and the voltage regulator 127 are all connected to the second output voltage VDD for power-on.

Referring to Fig. 1, the plurality of electricity-consuming components in the transmitter module 120 includes a sensor excitation unit 123, the sensor signal conditioning unit 124 and the processor unit 125. The sensor excitation unit 123 is connected to the aforementioned blood glucose sensor 112 and configured to generate an excitation voltage which is applied to the blood glucose sensor 112. The sensor signal conditioning unit 124 is configured to collect an output electrical signal from the blood glucose sensor 112, which may be in particular an analog current signal, condition the analog current signal and convert it into a corresponding analog voltage signal. In this embodiment, the blood glucose sensor 112 detects the interstitial fluid in the human body and outputs weak analog current signals to the sensor signal conditioning unit 124, the sensor signal conditioning unit 124 receives and processes the analog current signals and produces analog voltage signals corresponding to the collected analog current signals. The processor unit 125 is configured to receive and process an output analog voltage signal from the sensor signal conditioning unit 124, derive a measurement value reflecting a blood glucose level in the human body and send the measurement value to the terminal device. The processor unit 125 may also be configured to control the sensor excitation unit 123 to increase or decrease an excitation voltage that it applies to the blood glucose sensor 112. The processor unit 125 may be implemented as a microcontroller unit (MCU) chip.

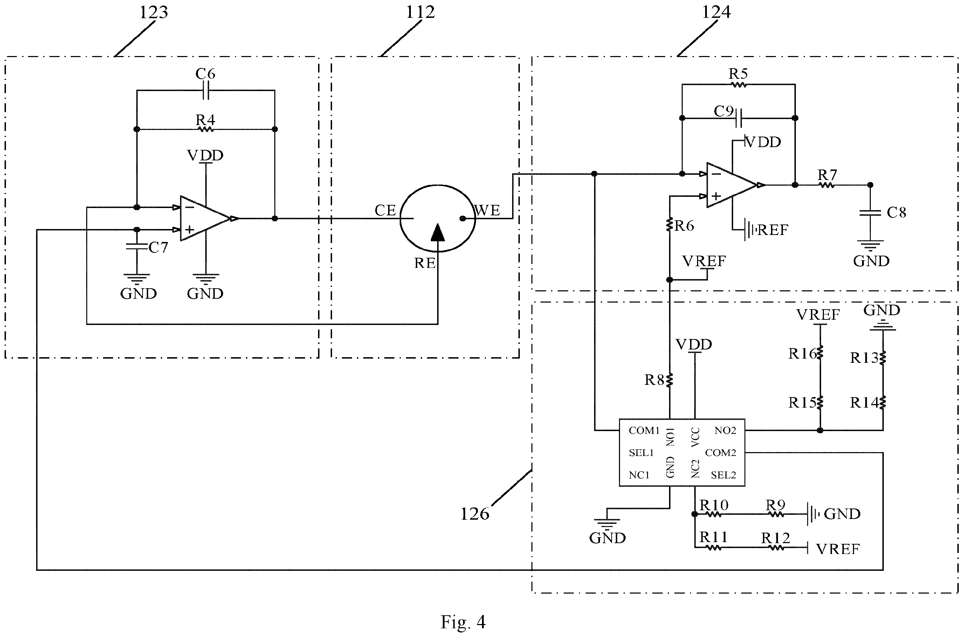

Fig. 4 is a schematic circuit diagram of the blood glucose sensor, the sensor excitation unit, the sensor signal conditioning unit and the bias voltage control unit in the BGM circuit according to an embodiment of this application. Referring to Fig. 4, for example, the sensor excitation unit 123 comprises a single-stage operational amplifier (op-amp) circuit. In this embodiment, the sensor excitation unit 123 is provided by, for example, a dual-channel op-amp chip (preferably with low power consumption, such as the model LPV802). Specifically, a first stage (channel) of the dual-channel op-amp chip constitutes a sensor excitation circuit of the sensor excitation unit 123 for applying an excitation voltage to the blood glucose sensor 112. The sensor excitation unit 123 further includes a low-pass filter network for eliminating AC signals exceeding a preset value (e.g., 0.8 Hz), which consists of a resistor R4 and a capacitor C6. Electrodes of the blood glucose sensor 112 are connected to the sensor excitation unit 123 by the aforementioned three contacts RE, CE, WE. In response to the sensor excitation unit 123 applying a suitable excitation voltage to the blood glucose sensor 112, the blood glucose sensor 112 starts operation to produce weak current signals based on a blood glucose concentration in the user, which reflect a blood glucose level in the human body.

Referring to Fig. 4, the sensor signal conditioning unit 124 comprises a single-stage op-amp circuit. In this embodiment, the sensor signal conditioning unit 124 is provided by the dual-channel op-amp chip with low power consumption that also provides the sensor excitation unit 123 (e.g., the model LPV802). Specifically, a second stage (channel) of the dual-channel op-amp chip constitutes a current-to-voltage (I/V) conversion circuit of the sensor signal conditioning unit 124 for converting a weak current (e.g., about 0-100 nA) output from the blood glucose sensor 112 into a corresponding voltage signal (e.g., about 0-50 mV) through a resistor R5. The resistor R5 and a capacitor C9 make up a low-pass filter network for filtering out high-frequency signals. An output part of the second-stage op-amp that constitutes the sensor signal conditioning unit 124 further includes a low-pass filter network for eliminating AC signals exceeding a preset value (e.g., 3 Hz), which consists of a resistor R7 and a capacitor C8.

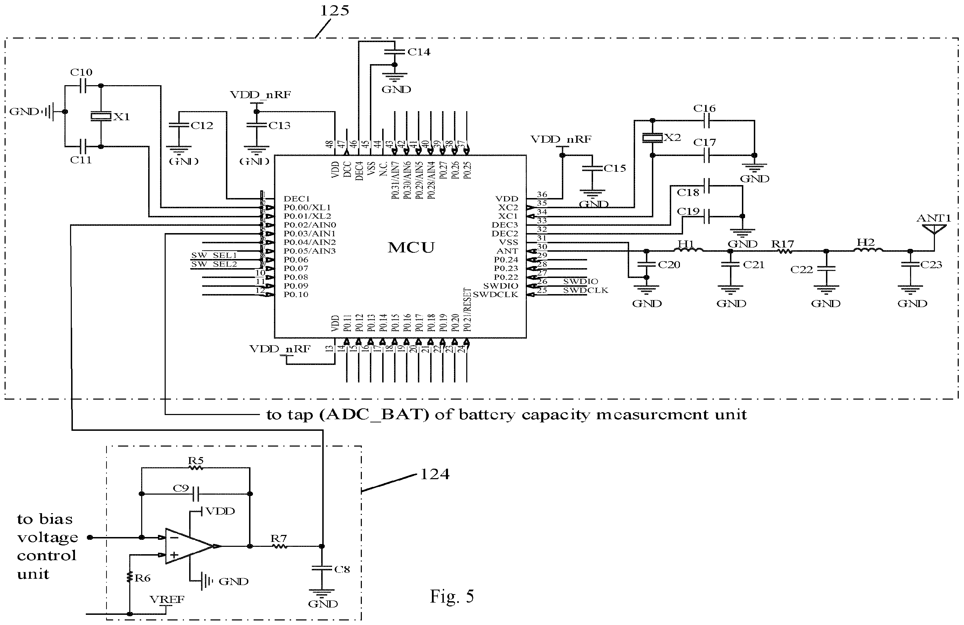

Fig. 5 is a schematic circuit diagram of the processor unit and the sensor signal conditioning unit in the BGM circuit according to an embodiment of this application. Referring to Fig. 5, the processor unit 125 is selected as, for example, an MCU chip incorporating an analog-to-digital converter (ADC) (preferably, a high-precision ADC), a filter and a wireless communication module (e.g., the model nRF52810). Reference can be made to the user manual of the MCU chip for more details of the chip elements and labels shown in Fig. 5. VDD nRF represents the aforementioned first output voltage. The ADC in the processor unit 125 is configured to amplify analog voltage signals output from the sensor signal conditioning unit 124 and convert the amplified signals into linearly corresponding digital voltage signals. The filter in the processor unit 125 is configured to perform filtering and smoothing processing on the digital voltage signals output from the ADC and thereby produce measurement values reflecting a blood glucose level in the human body. For example, in a BGM process, filtering and smoothing processing are performed on consecutively collected ten digital voltage signals, which involves discarding the maximum and minimum values and averaging the remaining values to produce a measurement value. In this embodiment, the measurement value is a voltage value. In order to enable corresponding blood glucose values to be directly read from the terminal device, before or after the wireless communication module (e.g., labeled as "Bluetooth 4.0" in Fig. 1) outputs corresponding signals to the terminal device (e.g., a mobile phone, computer, server, etc.), such measurement values are converted into corresponding current values, which are then substituted into an associated calculation formula to derive the blood glucose values.

For example, referring to Figs. 4 and 5, after the processor unit 125 receives the analog voltage signals output from the sensor signal conditioning unit 124, the ADC performs analog-to-digital conversion on the analog signals output from the sensor signal conditioning unit 124. For example, if an internal gain of the ADC is set to 6, a weak analog current signal in the range of 0-100 nA output from the blood glucose sensor 112 is converted by the sensor signal conditioning unit 124 into an analog voltage signal in the range of 0-50 mV, which is in turn converted by the ADC in the processor unit 125 into a linearly corresponding digital voltage signal in the range of 0-300 mV. After processed in the filter, the digital voltage signal indicates a measurement value. Subsequently, this valid voltage value is converted into a current value, which is then transmitted to the terminal device through the included wireless communication module.

Referring to Figs. 1 and 4, in this embodiment, the transmitter module 120 further includes the bias voltage control unit 126, the transmitter module 120 is configured to output different control signals to the sensor excitation unit 123 in different time intervals during operation of the blood glucose sensor 112 to modify the excitation voltage applied to the blood glucose sensor 112. For example, the excitation voltage may be configured to experience a drop upon the expiry of an activation time interval after the blood glucose sensor 112 is implanted into the human body. The bias voltage control unit 126 is connected to the aforementioned processor unit 125 and the sensor excitation unit 123. The bias voltage control unit 126 may output the control signals under the control of commands from the processor unit 125.

Referring to Fig. 4, for example, the bias voltage control unit 126 includes a voltage divider chip (e.g., the model TS2A5223) configured to divide the second output voltage VDD output from the battery 121. The NO2 and NC2 terminals of the voltage divider chip are respectively connected to a first voltage-dividing circuit and a second voltage-dividing circuit. With this arrangement, in different time intervals during operation of the blood glucose sensor 112, the bias voltage control unit 126 may select different ones of the first and second voltage-dividing circuits for voltage division and provide the divided voltages to the sensor excitation unit 123. For example, the first voltage-dividing circuit includes resistors R13, R14, R15, R16, in which R13 and R14 are connected in series between the NO2 terminal of the voltage divider chip and a ground terminal, and R15 and R16 are connected in series between the NC2 terminal of the voltage divider chip and the ground. The second voltage-dividing circuit includes resistors R9, R10, R11, R12, in which R9 and R10 are connected in series between the NC2 terminal of the voltage divider chip and a ground, and R11 and R12 are connected in series between the NC2 terminal of the voltage divider chip and a reference voltage VREF (which is provided by the voltage regulator as detailed below). The voltage divider chip may be switched between the first and second voltage-dividing circuits by means of an analog switch. In this way, it can obtain a divided voltage from only one of the first and second voltage-dividing circuits at any time.

Generation of divided voltages in different time intervals during operation of the blood glucose sensor 112 is exemplified below. In one example, within one hour after the sensor module 110 is implanted into the human body, which is defined as an activation time interval, the bias voltage control unit 126 is switched to the first voltage-dividing circuit under the control of a command from the processor unit 125, resulting in the generation of a first divided voltage, from which a corresponding output signal is produced and output to the sensor excitation unit 123. In response, the sensor excitation unit 123 generates an excitation voltage and applies it between the electrode terminals WE and RE of the blood glucose sensor 112. For example, the excitation voltage that is derived from the first divided voltage and applied between the electrode terminals WE and RE is 0.7 V. When one hour elapses after the sensor module 110 is implanted into the human body, the bias voltage control unit 126 is switched to the second voltage-dividing circuit under the control of a command from the processor unit 125, resulting in the generation of a second divided voltage, from which a corresponding output signal is produced and output to the sensor excitation unit 123. In response, the sensor excitation unit 123 generates an excitation voltage and applies it between the electrode terminals WE and RE of the blood glucose sensor 112. For example, the excitation voltage that is derived from the second divided voltage and applied between the electrode terminals WE and RE is 0.35 V. Advantageously, through providing different divided voltages in different time intervals during operation of the blood glucose sensor 112, a higher voltage can be provided within a certain period of time after the sensor module 110 is exposed to the interstitial fluid in the human body (which is a one-hour period in this embodiment, but is otherwise configured as required in practical applications) to result in a higher excitation voltage of, for example, 0.7 V (which may be otherwise configured as required in practical applications) between the electrode terminals WE and RE, which is required by hydration and activation. This higher excitation voltage can initiate electrochemical reactions around the probe and help them reach equilibrium. Moreover, it allows the time period to have an appropriate length which enables obtaining feedback of the blood glucose sensor 112 and hence measurements of blood glucose level in the human body as early as possible. In addition, a lower voltage (derived from a lower divided voltage output from the bias voltage control unit 126) can be provided when the period of time after the sensor module 110 is exposed to the interstitial fluid in the human body elapses to allow glucosidases around the probe to react at a reasonable speed which avoids them from being consumed too fast to ensure that the blood glucose sensor can effectively operate long enough (e.g., 14 days or longer). The lower excitation voltage derived from the lower divided voltage and applied between the electrode terminals WE and RE may be set to a value which can minimize interference from other components of the interstitial fluid, such as half that applied in the initial time interval, i.e., 0.35 V (which may be otherwise configured as required in practical applications).

Referring to Figs. 1 and 3, the electricity-consuming components in the transmitter module 120 may further include the voltage regulator 127, the voltage regulator 127 is connected to the aforementioned bias voltage control unit 126. The voltage regulator 127 is configured to generate the reference voltage based on an output of the battery 121 and provide the reference voltage to the bias voltage control unit 126. As shown in Fig. 3, for example, the voltage regulator 127 includes a voltage regulator chip Q1 (e.g., the model REF3312). The input (IN) of the voltage regulator chip is connected to an output node (VDD) of the aforementioned secondary output circuit, and the reference voltage VREF is output from its output (OUT) to the bias voltage control unit 126 as a reference voltage source for voltage division. The reference voltage VREF may also serve as a baseline for the analog voltage signals output from the bias voltage control unit 126. The voltage regulator 127 further includes a capacitor C4 arranged between the output node of the secondary output circuit and a ground and a capacitor C5 arranged between an output node of the reference voltage VREF and the ground. The capacitors C4 and C5 serve for power supply filtering.

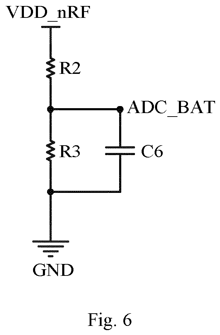

Referring to Fig. 1, the electricity-consuming components in the transmitter module 120 may further include the battery capacity measurement unit 128, the battery capacity measurement unit 128 is configured to measure remaining capacity of the battery 121 and feed it back to the processor unit 125. Fig. 6 is a schematic circuit diagram of the battery capacity measurement unit in the BGM circuit according to an embodiment of this application. As an example, the battery capacity measurement unit 128 divides an output voltage of the battery 121 (the output voltage of the battery 121 is for example the first output voltage VDD_nRF from the aforementioned primary output circuit) using a circuit consisting of resistors R2 and R3. Referring to Fig. 6, the resistors are connected in series between the first output voltage VDD nRF and a ground, and a divided voltage is obtained at a node where the resistors are connected in series to each other. In this way, a DC voltage ADC_BAT (e.g., 0.36-0.54 V) can be obtained by dividing the voltage of the battery 121 (e.g., 2-3 V). The DC voltage ADC_BAT is fed to the processor unit 125. As shown in Fig. 1, the processor unit 125 may include more than one ADC, and the DC voltage ADC BAT may be provided to another ADC in the processor unit 125 than the one that collects output signals from the blood glucose sensor 112, allowing determining the remaining capacity of the battery 121 in the transmitter module 120. In the battery capacity measurement unit 128, the capacitor C6 and the resistor R3 make up a low-pass filter network.

The above discussed BGM circuit can be used for both dynamic and static blood glucose detection. As the magnetically controlled switch based power-on strategy minimizes power loss of the transmitter module 120, it is particularly suited to dynamic blood glucose detection. Moreover, it can extend the service life of the transmitter module 120 (expectedly, to a period of time that is more than one times the service life of the blood glucose sensor) without increasing the volume of the transmitter module 120 (i.e., without using a larger capacity battery). The battery can be easily replaced when it runs out. Therefore, dynamic blood glucose monitoring can be achieved in a convenient, reliable and inexpensive way. Further, the transmitter module 120 in the BGM circuit is optimized to offers the following benefits. First, the bias voltage control unit 126 in the transmitter module 120 allows the excitation voltage applied to the blood glucose sensor 112 to be adjusted. In this way, taking into account the service life of a BGM device incorporating the BGM circuit, electrochemical reactions around the probe of the blood glucose sensor can be initiated and reach equilibrium more quickly, shortening an initial adaptation period required by the BGM device and allowing it to provide measurements faster. Moreover, glucosidases are allowed to react at a reasonable speed which avoids them from being consumed too fast to ensure that the blood glucose sensor can effectively operate long enough. Besides, interference from other components of the interstitial fluid can be minimized. Second, the use of the single-use disposable battery in the transmitter module 120 dispenses with a power supply management module and a charging interface, which would be otherwise necessary if a rechargeable battery is used. In this way, a reduced volume of the transmitter module and increased ease of user operation can be achieved. Third, the use of the processor unit 125 that incorporates the ADC and the wireless communication module with the bias voltage control unit 126 enables a higher degree of circuit integration. Fourth, the sensor excitation unit 123 and the sensor signal conditioning unit 124 in the transmitter module 120 are provided by a dual-channel op-amp chip, and the second output voltage VDD from the battery 121 is provided at a power supply terminal of the chip to achieve current limiting. Both these can help reduce power consumption of the chip and prolong the battery life of the transmitter module 120. As demonstrated by tests on a BGM device incorporating the BGM circuit discussed above, in which a lithium manganese coin cell was used as the battery 121 in the transmitter module 120, an average current output from the transmitter module 120 during normal operation (steady-state operation at 3 V), which reflected a blood glucose level in a human body, was about 35.6 µA, and the battery life of the transmitter module 120 operating in a dynamic BGM mode could be as long as 60 days or more.

Embodiments of this application also relate to a medical device including the BGM circuit as discussed above. The medical device is, for example, a BGM device, or a multifunctional medical device with BGM capabilities. In the medical device, the sensor module 110 and the transmitter module 120 of the BGM circuit can be connected to, or detached from, each other, and the BGM circuit can be used to obtain valid digital signals reflecting a blood glucose level in a human body. The medical device further includes at least one terminal device, and through the wireless communication module in the processor unit 125, the transmitter module 120 can transmit the valid digital signals reflecting the blood glucose level in the human body, or blood glucose data derived from the valid digital signals, to the terminal device. Moreover, the terminal device may convert the valid digital signals reflecting the blood glucose level in the human body that it receives to blood glucose values and provide them to a user for reference. The magnetically controlled switch in the BGM circuit can automatically initiate BGM of the user once the device is worn on him/her, providing ease of operation. Additionally, since the transmitter module 120 is optimized in terms of energy consumption and battery life, the medical device exhibits good performance.

The foregoing description is merely that of several preferred embodiments of this application and is not intended to limit the scope of thereof in any way. Any person of skill in the art may make various possible variations and changes to the disclosed embodiments in light of the methodologies and teachings disclosed hereinabove, without departing from the spirit and scope of the application. Accordingly, any and all such simple variations, equivalent alternatives and modifications made to the foregoing embodiments based on the essence of this application without departing from the scope of the embodiments are intended to fall within the scope of the application.

## Claims

1. A blood glucose monitoring circuit, comprising:
a sensor module comprising a blood glucose sensor, the blood glucose sensor configured to detect a blood glucose level in a human body and output a corresponding electrical signal; and
a transmitter module detachably connected to the sensor module, the transmitter module comprising a battery, a plurality of electricity-consuming components and a magnetically controlled switch disposed between the battery and the plurality of electricity-consuming components, the transmitter module configured to apply an excitation voltage to the blood glucose sensor, collect the electrical signal output from the blood glucose sensor and process the electrical signal to generate a measurement value reflecting the blood glucose level in the human body,
wherein the sensor module further comprises a magnet, and when the magnetically controlled switch approaches the magnet to a predetermined extent, a circuit of the magnetically controlled switch is closed, and the plurality of electricity-consuming components are powered on.

2. The blood glucose monitoring circuit of claim 1, wherein the plurality of electricity-consuming components in the transmitter module comprise:
a sensor excitation unit for generating the excitation voltage applied to the blood glucose sensor;
a sensor signal conditioning unit for collecting the electrical signal output from the blood glucose sensor, wherein the electrical signal is an analog current signal, and converting the analog current signal to a corresponding analog voltage signal; and
a processor unit for receiving the analog voltage signal output from the sensor signal conditioning unit, generating the measurement value reflecting the blood glucose level in the human body by processing the analog voltage signal and sending the measurement value to a terminal device.

3. The blood glucose monitoring circuit of claim 2, wherein the processor unit comprises:
an analog-to-digital converter for amplifying the analog voltage signal output from the sensor signal conditioning unit and converting the amplified signal into a linearly corresponding digital voltage signal;
a filter for generating the measurement value reflecting the blood glucose level in the human body by performing filtering and smoothing processing on the digital voltage signal output from the analog-to-digital converter; and
a wireless communication module for transmitting the measurement value to the terminal device.

4. The blood glucose monitoring circuit of claim 2, wherein the sensor excitation unit and the sensor signal conditioning unit are provided by a dual-channel operational amplifier chip.

5. The blood glucose monitoring circuit of claim 1, wherein the plurality of electricity-consuming components in the transmitter module comprise a bias voltage control unit and a sensor excitation unit, the bias voltage control unit configured to output, in different time intervals during operation of the blood glucose sensor, respective control signals to the sensor excitation unit to adjust the excitation voltage applied to the blood glucose sensor.

6. The blood glucose monitoring circuit of claim 5, wherein the bias voltage control unit comprises a voltage divider chip, a first voltage-dividing circuit and a second voltage-dividing circuit, both the first voltage-dividing circuit and the second voltage-dividing circuit connected to the voltage divider chip, the voltage divider chip switchable between the first voltage-dividing circuit and the second voltage-dividing circuit by means of an analog switch so that one of the first voltage-dividing circuit and the second voltage-dividing circuit divides a voltage and produces a corresponding divided voltage, and the divided voltage is then output to the sensor excitation unit.

7. The blood glucose monitoring circuit of claim 5, wherein the excitation voltage is configured as: relative to an activation time interval for the blood glucose sensor is implanted into the human body, a value of the excitation voltage decreases after the blood glucose sensor is implanted into the human body upon the expiry of the activation time interval.

8. The blood glucose monitoring circuit of claim 6, wherein the plurality of electricity-consuming components in the transmitter module further comprise:
a voltage regulator connected to the bias voltage control unit, the voltage regulator configured to generate a reference voltage based on an output from the battery and provide the reference voltage to the bias voltage control unit as a reference voltage source for voltage division; and/or
a battery capacity measurement unit for measuring remaining capacity of the battery which is fed back to the processor unit.

9. The blood glucose monitoring circuit of claim 8, wherein the transmitter module comprises a primary output circuit and a secondary output circuit, the primary output circuit and the secondary output circuit connected in sequence to an output of the battery, the primary output circuit comprising an output node arranged downstream of the magnetically controlled switch and upstream of the plurality of electricity-consuming components, an output voltage of the primary output circuit exceeding an output voltage of the secondary output circuit, wherein the processor unit and the battery capacity measurement unit are both connected to the output voltage of the primary output circuit for power-on, and the sensor signal conditioning unit, the bias voltage control unit and the voltage regulator are all connected to, and powered by, the output voltage of the secondary output circuit.

10. The blood glucose monitoring circuit of any one of claims 1 to 9, wherein the battery is implemented as a single-use disposable battery.

11. A medical device, comprising the blood glucose monitoring circuit of any one of claims 1 to 10.
